# EUROPÄISCHE PATENTSCHRIFT

(11) **EP 1 284 718 B1**
(45) Veröffentlichungstag und Bekanntmachung des Hinweises auf die Patenterteilung: **29.03.2006**
(21) Anmeldenummer: 01981915.0
(22) Anmeldetag: 15.05.2001
(51) Int. Cl.: A61K 9/70

(54) **OKKLUSIVES TRANSDERMALES THERAPEUTISCHES SYSTEM MIT NICHT-OKKLUSIVER RÜCKSCHICHT**
OCCLUSIVE TRANSDERMAL THERAPEUTIC SYSTEM WIT A NON-OCCLUSIVE BACKING LAYER
SYSTEME THERAPEUTIQUE OCCLUSIF POURVU D'UNE COUCHE DORSALE NON OCCLUSIVE

(30) Priorität: 31.05.2000 DE 10027258
(43) Veröffentlichungstag der Anmeldung: 26.02.2003
(73) Patentinhaber: LTS LOHMANN Therapie-Systeme AG, 56626 Andernach (DE)
(72) Erfinder: BRACHT, Stefan, 07751 Cospeda (DE); SCHMITZ, Christoph, 56598 Rheinbrohl (DE)
(74) Vertreter: Schmidt, Werner
(86) Internationale Anmeldenummer: PCT/EP2001/005474
(87) Internationale Veröffentlichungsnummer: WO 2001/091718

(56) Entgegenhaltungen:
- EP-A- 0 739 626
- WO-A-00/64419
- US-A- 4 746 509
- US-A- 5 989 586

## Beschreibung

Die Verabreichung von Wirkstoffen durch die Haut wird in der Mehrzahl der Fälle durch die geringe Durchlässigkeit der Haut erschwert. Daher müssen häufig alle zur Verfügung stehenden Möglichkeiten zur Steigerung der Durchlässigkeit ausgeschöpft werden. Die Aufnahme von Wirkstoffen durch die Haut wird in praktisch allen Fällen durch den Effekt der Okklusion begünstigt. Hierunter ist ein Wasserdampfstau in den oberen Hautschichten zu verstehen, wie er nach Applikation von transdermalen therapeutischen Systemen (TTS) entsteht, die mindestens eine wasserdampf-undurchlässige Schicht enthalten.

Als wasserdampfundurchlässige Schicht eines TTS eignet sich vor allem dessen Rückschicht. Aus diesem Grunde ist die Verwendung wasserdampfundurchlässiger Rückschichten Stand der Technik und bei fast allen Marktprodukten anzutreffen.

Typischerweise werden dünne Kunststofffolien aus Polyethylenterephtalat (PET) verwendet. Gerade diese wasserdampfsperrenden Folien sind jedoch zumeist unelastische, starre Materialien, die sich der Haut wenig anzuschmiegen vermögen und Dehnungen oder Stauchungen der beklebten Hautoberfläche nicht in physiologischer Weise zulassen. Damit verbunden ist ein geringer Tragekomfort für den Patienten, insbesondere bei größeren TTS ab etwa 25 cm² Fläche. Mechanisch besonders beanspruchte Körperpartien wie die großen Gelenke der Extremitäten können mit solchen Produkten, unter Erhalt der vollen Beweglichkeit, praktisch überhaüpt nicht dauerhaft beklebt werden.

Schließlich wird bei einem TTS mit einer solchen starren Rückschicht die Tragedauer dadurch verkürzt, daß die mechanische Inkompatibilität zwischen elastischer Haut und unelastischem TTS eine rasche Ablösung der letzteren zur Folge hat: Die andauernden mechanischen Spannungen können von der Klebeschicht nicht dauerhaft gehalten werden. Elastische Folien, die in dieser Hinsicht vorteilhafter wären, etwa aus Polyurethan oder Ethylenvinylacetat (EVA) sind hingegen wieder gut wasserdampfdurchlässig und erzeugen nur geringe Okklusion, wenn überhaupt.

Gewebe oder Fliesstoffe schließlich bewirken als Rückschichten praktisch keine Okklusion, die für die Durchlässigkeit der Haut von Bedeutung sein könnte. Alternativ kann nun eine der anderen Schichten eines TTS wasserdampfsperrend ausgebildet werden, um den Effekt der Okklusion auch bei einer wasserdampfdurchlässigen Rückschicht nutzen zu können.

Im Bereich der Haftkleberschichten eignen sich dazu insbesondere Formulierungen auf der Basis von reinen Kohlenwasserstoffpolymeren.

Dabei handelt es sich jedoch um sehr lipophile Polymere, die typischerweise ein geringes Lösungsvermögen für pharmazeutische Wirkstoffe aufweisen. Diese können dann häufig nur in weitgehend ungelöster Form, z. B. als Kristalldispersion eingebettet werden oder müssen überhaupt in einer zusätzlichen, anders formulierten Schicht untergebracht werden.

Ungelöste Wirkstoffe ergeben in der Regel unbefriedigendes Freigabeverhalten aus dem TTS, zusätzliche Schichten verkomplizieren und verteuern den Systemaufbau.

In dieser Hinsicht kann nach dem Stand der Technik der Effekt der Okklusion bei TTS mit einer elastischen Rückschicht, insbesondere einem elastischen Gewebe, nur teilweise oder gar nicht genutzt werden.

Aufgabe der vorliegenden Erfindung ist es daher, ein transdermales therapeutisches System mit einer elastischen Rückschicht und einer wirkstoffhaltigen Schicht, welche wasserdampfsperrende Eigenschaften hat, zu entwickeln.

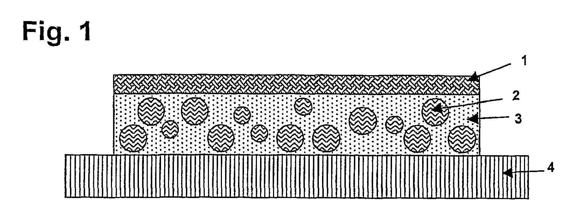

Diese Aufgabe wird durch einen überraschend übersichtlichen und effizienten Systemaufbau gelöst (Fig. 1).

Der erfindungsgemäße System besteht im wesentlichen aus einer wirkstoffhaltigen Klebeschicht, die jedoch zweiphasig ausgeführt wird.

In die äußere, wasserdampfsperrende Phase (3) wird eine innere Phase (2), die den Wirkstoff in gelöster Form enthält, dispers eingebettet. In Verbindung mit einer elastischen Rückschicht (1), vorzugsweise einem längs-quer-elastischen Gewebe, ergibt sich ein sehr dünnes Matrixsystem mit ausgezeichnetem Tragekomfort bei gleichzeitig optimaler Ausnutzung der Okklusion zur gesteigerten Wirkstoffaufnahme über die Haut.

Zur Abdeckung der haftklebenden Oberfläche bis zur Anwendung ist standardmäßig eine wiederablösbare Schutzfolie (4) aus herkömmlichem Material, z. B. silikonisiertem Polyethylenterephthalat (PET) vorgesehen.

Als geeignete Komponenten für die äußere und innere Phase der haftklebenden Matrix kommen im Hinblick auf die lösungsmittelbasierte Verarbeitung Komponenten in Frage, die in Lösung eine stabile Emulsion ergeben und auch nach Beschichtung und Trocknung (Entfernung von Prozeßlösemitteln) ein stabiles, zweiphasiges System bilden.

Für die äußere Phase werden vorzugsweise Polymere ans der Gruppe der Polyisobutylene, Polyisopren, Polybutene und der Styrol-Blockpolymere mit Isopren oder Butadien verwendet. Diese haben wasserdampfsperrende Eigenschaften und eignen sich bei Vermischung verschiedener Typen mit unterschiedlichen Molekulargewichten als Haftkleber.

Die innere Phase kann gebildet werden aus der Lösung des Wirkstoffs in geeigneten flüssigen Hilfsstoffen oder aber aus einer Lösung des Wirkstoffs in einem oder mehreren Polymeren.

Die Lösung in einem Polymer ist vorzuziehen, da tröpfchenartig dispergierte Lösungen als innere Phase häufig zum Ausschwitzen oder zum Ausbluten bei mechanischer Beanspruchung des tröpfchenhaltigen Filmes neigen.

Polymere, die für die Lösung des Wirkstoffs in Betracht kommen, sollten kompatibel mit den oben für die äußere Phase als geeignet genannten Polymeren sein. Dabei ist unter Kompatibilität zu verstehen, daß in der 2-phasigen Mischung besonders niedrige Grenzflächenenergien auftreten, die sich in einem möglichst hohen Dispersionsgrad und geringer Entmischungstendenz der Emulsion äußern. Als sehr kompatibel in diesem Sinne haben sich Acrylatpolymere und Methacrylatcopolymere und Ethylvinylacetatcopolymere erwiesen.

Bei den (Meth)Acrylatcopolymeren kann es sich auch um ihrerseits haftklebende Polymere handeln. Eingebettet in eine äußere Haftklebephase aus Kohlenwasserstoffpolymeren resultiert dann eine in ihren viskoelastischen Eigenschaften weitgehend einheitliche Schicht, was sich auf die Trageeigenschaften auf der Haut positiv auswirken kann.

Unter den (Meth)Acrylatcopolymeren können solche Typen von Vorteil sein, die freie Carboxylgruppen enthalten. Durch Neutralisation dieser Gruppen mit geeigneten alkalischen Hilfsstoffen, z. B. Kaliumhydroxid, kann die

Hydrophilie/Lipophilie-Balance eines solchen Polymers individuell eingestellt werden. Dies kann für die Einstellung einer stabilen Emulsion im Gemisch mit Kohlenwasserstoffpolymeren vorteilhaft sein.

Als Wirkstoffe kommen insbesondere nicht-steroidale Antiphlogistika (dt. NSAR für nicht-steriodales Antirheumatikum, engl. NSAID für non-steriodal anti inflammatory drug) in Betracht. Diese werden häufig lokal äußerlich angewendet im Bereich von Gelenken, insbesondere der Extremitäten. Gerade an diesen mechanisch stark beanspruchten Applikationsorten erweisen sich die erfindungsgemäßen TTS als besonders vorteilhaft. Ohne Anspruch auf Vollständigkeit handelt es sich um Wirkstoffe aus der Gruppe von Diclofenac oder einem seiner pharmazeutisch akzeptablen Salze, vorzugsweise dem Natriumsalz, Ibuprofen, Ketoprofen, Fluriprofen, Etofenamat, Hydroxyethylsalicylat, Meloxicam, Piroxicam, Lornoxicam, Tenoxicam oder Indometacin.

Neben den Polymeren für die äußere Phase, gegebenenfalls Polymeren für die innere Phase und dem pharmazeutischen Wirkstoff können noch zahlreiche andere Hilfsstoffe zum Einsatz kommen, wie sie für die Verwendung in TTS der Fachwelt bekannt sind.

So können z. B. Permeationsverstärker, vorzugsweise in der inneren Phase der Matrix, eingesetzt werden. Als Permeationsverstärker kommen in Frage Verbindungen aus der Gruppe der niedermolekularen ein- oder mehrwertigen Alkoholen, Fettsäuren (vorzugsweise Ölsäure), Fettalkoholen, Fettalkoholethern, polyoxyethylierten Fettalkoholen, Fettsäureestern (speziell Monoglyceride und Monoester mit Propylenglykol), Sorbitanfettsäureestern und polyoxyethylierten Sorbitanfettsäurestern, sowie Dimethylisosorbit.

Ferner kommen in Frage grenzflächenaktive Tenside, die die Stabilität der zweiphasigen Matrixschicht positiv zu beeinflussen vermögen, indem sie die Grenzflächenenergie herabsetzen.

Als elastische, wasserdampfdurchlässige Rückschichten kommen unter anderem Folien aus Polyurethan oder Ethylvinylacetatcopolymere in Frage. Insbesondere sind jedoch Gewebe oder nicht-gewebte Fliesstoffe, bzw. Verbunde aus solchen geeignet. Als Materialien kommen hier z. B. Baumwolle, Cellulose, Viskose, Polyurethan oder Polyethylenterephthalat (PET) in Betracht.

Ganz besonders geeignet sind längs- und querelastische Gewebe aus PET.

### Beispiel 1

Das erfindungsgemäße transdermale therapeutische System besitzt folgenden Aufbau bzw. Zusammensetzung (Gew.-%):

| Innere Phase: | |
|---|---|
| Ketoprofen | 4,00 % |
| Ölsäure | 4,00 % |
| Kaliumhydroxid | 0,58 % |
| Aluminiumionen | 0,008 % |
| Durotak 387-2353 | 11,4 % |

| Äußere Phase: | |
|---|---|
| Oppanol B10 | 60,0 % |
| Oppanol B100 | 20,0 % |

Als Rückschicht dient ein bielastisches Gewebe aus PET.

### Beispiel 2 (Vergleichsbeispiel)

Das transdermale therapeutische System mit üblichem einphasigen Aufbau hat die folgende Zusammensetzung (Gew.-%):

| | |
|---|---|
| Ketoprofen | 10,00 % |
| Ölsäure | 18,00 % |
| Durotak 387-2052 | 72,00 % |

Als Rückschicht dient dasselbe längsquerelastische PET-Gewebe wie für das erfindungsgemäße Beispiel 1.

Zur Herstellung werden Ketoprofen und Ölsäure in der Kleberlösung Durotak (National Starch & Chemical) durch Einrühren homogen gelöst bzw. verteilt. Die erhaltene einphasige Lösung wird auf eine wieder ablösbare Trägerfolie aus silikonisiertem Polyethylenterephthalat (PET, 100µm Dicke) beschichtet und 10 Minuten lang in einem Abluftofen bei 80°C getrocknet. Das angestrebte Flächengewicht des getrockneten Kleberfilms beträgt 80 g/m².

Der getrocknete Kleberfilm wird mit einem längsquerelastischen PET-Gewebe (100 g/m² Flächengewicht) kaschiert.

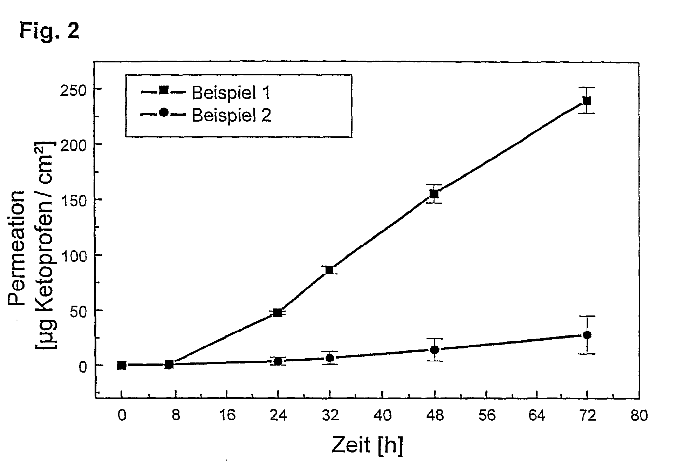

Fig. 2 zeigt in graphischer Form einen Vergleich der Wirkstoffpermeation an menschlicher Haut in vitro (volle Hautdicke, n=3, Experimente mit Haut vom selben Donor). Das nach dem erfindungsgemäßen Beispiel 1 hergestellte TTS erzielt sehr viel höhere Abgaberaten als ein nach dem Beispiel 2 hergestelltes konventionelles TTS, obwohl die Gesamtmenge des angebotenen Wirkstoffs (Ketoprofen) beim erfindungsgemäßen TTS sogar erheblich niedriger liegt als beim Vergieichs-TTS.

## Patentansprüche

1. Okklusives transdermales therapeutisches System, bestehend aus einer wasserdampf- und luftdurchlässigen elastischen Rückschicht, einer Matrixschicht, welche einen oder mehrere Wirkstoffe, ggf. einen oder mehrere Hilfsstoffe und ggf. einen oder mehrere Permeationsverstärker enthält, und einer wiederablösbaren Schutzschicht, **dadurch gekennzeichnet, daß** die Matrixschicht zwei-phasig ist und aus einer äußeren, weitgehend wasserdampf- und luftundurchlässigen Phase besteht, in welche eine den Wirkstoff enthaltende innere Phase dispers eingebettet ist.

2. Okklusives transdermales therapeutisches System nach Anspruch 1, **dadurch gekennzeichnet, daß** die elastische Rückschicht aus einem längs- und querelastischen Gewebe besteht.

3. Okklusives transdermales therapeutisches System nach Anspruch 2, **dadurch gekennzeichnet, daß** das Gewebe aus Polyethylenterephthalat (PET) besteht.

4. Okklusives transdermales therapeutisches System nach einem oder mehreren der vorangehenden Ansprüche, **dadurch gekennzeichnet, daß** die wasserdampf- und luftundurchlässige äußere Phase der Matrixschicht aus einem oder mehreren Kohlenwasserstoffpolymeren besteht.

5. Okklusives transdermales therapeutisches System nach einem oder mehreren der vorangehenden Ansprüche, **dadurch gekennzeichnet, daß** die innere Phase der Matrix den Wirkstoff in gelöster Form enthält.

6. Okklusives transdermales therapeutisches System nach Anspruch 5, **dadurch gekennzeichnet, daß** der Wirkstoff in geeigneten flüssigen Hilfsstoffen oder in einem oder mehreren Polymeren gelöst ist.

7. Okklusives transdermales therapeutisches System nach Anspruch 4, **dadurch gekennzeichnet, daß** die äußere Phase der Matrix aus einem oder mehreren Polymeren aus der Gruppe der Polyisobutylene, Polyisopren, Polybutene oder einem Styrol-Blockpolymeren mit Isopren oder Butadien besteht.

8. Okklusives transdermales therapeutisches System nach Anspruch 7, **dadurch gekennzeichnet, daß** die äußere Phase der Matrix aus Polyisobutylenen von mindestens zwei, vorzugsweise drei verschiedenen Molekulargewichten besteht.

9. Okklusives transdermales therapeutisches System nach einem oder mehreren der vorangehenden Ansprüche, **dadurch gekennzeichnet, daß** die den Wirkstoff in gelöster Form enthaltende innere Phase der Matrix aus einem oder mehreren Polymeren aus der Gruppe der Acrylatcopolymeren, Methacrylatcopolymeren oder Ethylvinylacetatcopolymeren besteht.

10. Okklusives transdermales therapeutisches System nach Anspruch 9, **dadurch gekennzeichnet, daß** es sich im Falle von Acrylat- oder Methacylatcopolymeren um carboxylgruppenhaltige Polymere handelt, die vorzugsweise zu 50-100 % neutralisiert als Natrium- oder Kaliumsalze vorliegen.

11. Okklusives transdermales therapeutisches System nach einem oder mehreren der vorangehenden Ansprüche, **dadurch gekennzeichnet, daß** die innere Phase der Matrix frei von Polymeren ist und den Wirkstoff in mindestens einem flüssigen Hilfsstoff gelöst enthält.

12. Okklusives transdermales therapeutisches System nach einem oder mehreren der vorangehenden Ansprüche, **dadurch gekennzeichnet, daß** der Anteil der inneren Phase der Matrix 5-40 Gew.-%, vorzugsweise 10-25 Gew.-% der wirkstoffhaltigen Matrix beträgt.

13. Okklusives transdermales therapeutisches System nach einem oder mehreren der vorangehenden Ansprüche, **dadurch gekennzeichnet, daß** die wirkstoffhaltige Matrix eine Schichtdicke von 60-200 g/m², vorzugsweise 80-120 g/m² beträgt.

14. Okklusives transdermales therapeutisches System nach einem oder mehreren der vorangehenden Ansprüche, **dadurch gekennzeichnet, daß** die innere Phase in der äußeren Phase der Matrix eine Dispersion mit einem Tröpfchendurchmesser von 10 nm bis 10 µm, vorzugsweise von 100 nm bis 1 µm bildet.

15. Okklusives transdermales therapeutisches System nach einem oder mehreren der vorangehenden Ansprüche, **dadurch gekennzeichnet, daß** es einen oder mehrere Wirkstoffe aus der Gruppe der nicht-steroidalen Antiphlogistika enthält.

16. Okklusives transdermales therapeutisches System nach Anspruch 15, **dadurch gekennzeichnet, daß** es Diclofenac oder eines seiner pharmazeutisch akzeptablen Salze, Ibuprofen, Ketoprofen, Flurbiprofen, Etofenamat, Hydroxyethylsalicylat, Meloxicam, Piroxicam, Lornoxicam, Tenoxicam und/oder Indometacin enthält.

17. Okklusives transdermales therapeutisches System nach einem oder mehreren der vorangehenden Ansprüche, **dadurch gekennzeichnet, daß** der oder die Wirkstoffe in der inneren Phase der Matrix in einem Konzentrationsbereich von 50-150 Gew.-% der Sättigungslöslichkeit des Wirkstoffes oder der Wirkstoffe vorliegen.

18. Okklusives transdermales therapeutisches System nach einem oder mehreren der vorangehenden Ansprüche, **dadurch gekennzeichnet, daß** es mindestens einem Permeationsverstärker, vorzugsweise in der inneren Phase der Matrix enthält.

19. Okklusives transdermales therapeutisches System nach Anspruch 18, **dadurch gekennzeichnet, daß** der oder die Permeationsverstärker niedermolekulare ein- oder mehrwertige Alkohole, Fettsäuren, Fettalkohole, Fettalkoholether, polyoxyethylierte Fettalkohole, Fettsäureester, Sorbitanfettsäureester, polyoxyethylierte Sorbitanfettsäurester und/oder Dimethylisosorbit sind.

## Claims

1. An occlusive transdermal therapeutic system composed of a water-vapor-permeable and air-permeable elastic backing layer, a matrix layer which comprises one or more active substances, one or more auxiliaries if desired, and one or more permeation enhancers if desired, and a redetachable protective layer, **characterized in that** the matrix layer is in two phases and is composed of an outer phase, which is substantially impermeable to water vapor and air, and in which an inner phase comprising the active substance is dispersely embedded.

2. The occlusive transdermal therapeutic system of claim 1, **characterized in that** the elastic backing layer is composed of a longitudinally and transversely elastic woven.

3. The occlusive transdermal therapeutic system of claim 2, **characterized in that** the woven is composed of polyethylene terephthalate (PET).

4. The occlusive transdermal therapeutic system of one or more of the preceding claims, **characterized in that** the water-vapor-impermeable and air-impermeable outer phase of the matrix layer is composed of one or more hydrocarbon polymers.

5. The occlusive transdermal therapeutic system of one or more of the preceding claims, **characterized in that** the inner phase of the matrix contains the active substance in dissolved form.

6. The occlusive transdermal therapeutic system of claim 5, **characterized in that** the active substance is in solution in suitable liquid auxiliaries or in one or more polymers.

7. The occlusive transdermal therapeutic system of claim 4, **characterized in that** the outer phase of the matrix is composed of one or more polymers from the group of polyisobutylenes, polyisoprene, polybutenes or a styrene block polymer with isoprene or butadiene.

8. The occlusive transdermal therapeutic system of claim 7, **characterized in that** the outer phase of the matrix is composed of polyisobutylenes of at least two, preferably three, different molecular weights.

9. The occlusive transdermal therapeutic system of one or more of the preceding claims, **characterized in that** the inner phase of the matrix, containing the active substance in dissolved form, is composed of one or more polymers from the group of acrylate copolymers, methacrylate copolymers or ethyl-vinyl acetate copolymers.

10. The occlusive transdermal therapeutic system of claim 9, **characterized in that** in the case of acrylate or methacylate copolymers the polymers in question are carboxyl-containing polymers which are preferably in 50-100% neutralized form as sodium or potassium salts.

11. The occlusive transdermal therapeutic system of one or more of the preceding claims, **characterized in that** the inner phase of the matrix is free from polymers and contains the active substance in solution in at least one liquid auxiliary.

12. The occlusive transdermal therapeutic system of one or more of the preceding claims, **characterized in that** the fraction of the inner phase of the matrix is 5-40% by weight, preferably 10-25% by weight of the matrix comprising active substance.

13. The occlusive transdermal therapeutic system of one or more of the preceding claims, **characterized in that** the matrix comprising active substance amounts to a layer thickness of 60-200 g/m², preferably 80-120 g/m².

14. The occlusive transdermal therapeutic system of one or more of the preceding claims, **characterized in that** the inner phase forms a dispersion in the outer phase of the matrix, with a droplet diameter of from 10 nm to 10 *µ*m, preferably from 100 nm to 1 *µ*m.

15. The occlusive transdermal therapeutic system of one or more of the preceding claims, **characterized in that** it comprises one or more active substances from the group of non-steroidal antiinflammatory drugs.

16. The occlusive transdermal therapeutic system of claim 15, **characterized in that** it comprises diclofenac or one of its pharmaceutically acceptable salts, ibuprofen, ketoprofen, flurbiprofen, etofenamate, hydroxyethyl salicylate, meloxicam, piroxicam, lornoxicam, tenoxicam and/or indomethacin.

17. The occlusive transdermal therapeutic system of one or more of the preceding claims, **characterized in that** the active substance or substances is or are present in the inner phase of the matrix in a concentration range of 50-150% by weight of the saturation solubility of the active substance or of the active substances.

18. The occlusive transdermal therapeutic system of one or more of the preceding claims, **characterized in that** it comprises at least one permeation enhancer, preferably in the inner phase of the matrix.

19. The occlusive transdermal therapeutic system of claim 18, **characterized in that** the permeation enhancer or enhancers are low molecular mass monohydric or polyhydric alcohols, fatty acids, fatty alcohols, fatty alcohol ethers, polyoxyethylated fatty alcohols, fatty acid esters, sorbitan fatty acid esters, polyoxyethylated sorbitan fatty acid esters and/or dimethyl-isosorbitol.

## Revendications

1. Système thérapeutique transdermique occlusif constitué par une couche de fond élastique, perméable à la vapeur d'eau et à l'air, une couche matrice, qui renferme un ou plusieurs principes actifs, éventuellement une ou plusieurs matières auxiliaires et éventuellement un ou plusieurs activateurs de perméation, et une couche protectrice détachable, **caractérisé en ce que** la couche matrice est biphasique et est constituée par une phase externe essentiellement imperméable à la vapeur d'eau et à l'air, dans laquelle est noyée dispersée une phase interne renfermant le principe actif.

2. Système thérapeutique transdermique occlusif selon la revendication 1, **caractérisé en ce que** la couche de fond élastique est constituée par un tissu élastique en longueur et en largeur.

3. Système thérapeutique transdermique occlusif selon la revendication 2, **caractérisé en ce que** le tissu est constitué par le poly(téréphtalate d'éthylène) (PET).

4. Système thérapeutique transdermique occlusif selon une ou plusieurs des revendications précédentes, **caractérisé en ce que** la phase externe imperméable à la vapeur d'eau et à l'air de la couche matrice est constituée par un ou plusieurs polymères hydrocarbonés.

5. Système thérapeutique transdermique occlusif selon une ou plusieurs des revendications précédentes, **caractérisé en ce que** la phase interne de la matrice renferme le principe actif sous forme dissoute.

6. Système thérapeutique transdermique occlusif selon la revendication 5, **caractérisé en ce que** le principe actif est dissous dans des matières auxiliaires liquides appropriées ou dans un ou plusieurs polymères.

7. Système thérapeutique transdermique occlusif selon la revendication 4, **caractérisé en ce que** la phase externe de la matrice est constituée par un ou plusieurs polymères pris parmi le polyisobutylène, le polyisoprène, le polybutène ou un copolymère à blocs de styrène avec l'isoprène ou le butadiène.

8. Système thérapeutique transdermique occlusif selon la revendication 7, **caractérisé en ce que** la phase externe de la matrice est constituée par des polyisobutylènes présentant d'au moins deux, de préférence trois, poids moléculaires différents.

9. Système thérapeutique transdermique occlusif selon une ou plusieurs des revendications précédentes, **caractérisé en ce que** la phase interne de la matrice qui renferme le principe actif sous forme dissoute est constituée par un ou plusieurs polymères pris parmi les copolymères d'acrylates, les copolymères de méthacrylates ou les copolymères d'acétates d'éthyl-vinyle.

10. Système thérapeutique transdermique occlusif selon la revendication 9, **caractérisé en ce qu'**il s'agit dans le cas de copolymères d'acrylate ou de méthacrylate de polymères comprenant des groupes carboxyle qui se présentent sous forme de sels de sodium ou de potassium de préférence neutralisés de 50 à 100 %.

11. Système thérapeutique transdermique occlusif selon une ou plusieurs des revendications précédentes, **caractérisé en ce que** la phase interne de la matrice est exempte de polymères et renferme le principe actif dissous dans au moins une matière auxiliaire liquide.

12. Système thérapeutique transdermique occlusif selon une ou plusieurs des revendications précédentes, **caractérisé en ce que** le taux de la phase interne de la matrice est de 5 à 40 % en masse, de préférence de 10 à 25 % en masse, de la matrice renfermant le principe actif.

13. Système thérapeutique transdermique occlusif selon une ou plusieurs des revendications précédentes, **caractérisé en ce que** la matrice qui renferme le principe actif présente une épaisseur de couche de 60 à 200 g/m², de préférence de 80 à 120 g/m².

14. Système thérapeutique transdermique occlusif selon une ou plusieurs des revendications précédentes, **caractérisé en ce que** la phase interne dans la phase externe de la matrice forme une dispersion comprenant des gouttelettes d'un diamètre de 10 nm à 10 µm, de préférence de 100 nm à 1 µm.

15. Système thérapeutique transdermique occlusif selon une ou plusieurs des revendications, précédentes, **caractérisé en ce qu'**il renferme un ou plusieurs principes actifs du groupe des anti-inflammatoires non-stéroïdaux.

16. Système thérapeutique transdermique occlusif selon la revendication 15, **caractérisé en ce qu'**il renferme le diclophénac ou un de ses sels pharmaceutiquement acceptables, l'ibupropfène, le kétoprofène, le flurbiprofène, l'étofénamate, le salicylate d'hydroxyéthyle, le meloxicam, le piroxicam, le lornoxicam, le tenoxicam et/ou l'indométacine.

17. Système thérapeutique transdermique occlusif selon une ou plusieurs des revendications précédentes, **caractérisé en ce que** le ou les principes actifs dans la phase interne de la matrice sont présents dans un domaine de concentrations de 50 à 150 % en masse de la solubilité de saturation du principe actif ou des principes actifs.

18. Système thérapeutique transdermique occlusif selon une ou plusieurs des revendications précédentes, **caractérisé en ce qu'**il renferme au moins un activateur de perméation, de préférence dans la phase interne de la matrice.

19. Système thérapeutique transdermique occlusif selon la revendications 18, **caractérisé en ce que** le ou les activateurs de perméation sont les esters d'acides gras, les esters du sorbitane et les acides gras, les esters du sorbitane et les acides gras polyéthoxylés et/ou le diméthylisosorbitol, les acides gras, les alcools gras, les éthers d'alcools gras, les alcools gras polyéthoxylés, les alcools mono- et polyfonctionnels de bas poids moléculaire.
